# EUROPÄISCHE PATENTSCHRIFT

(11) **EP 4 144 384 B1**
(45) Veröffentlichungstag und Bekanntmachung des Hinweises auf die Patenterteilung: **09.10.2024**
(21) Anmeldenummer: 21194251.1
(22) Anmeldetag: 01.09.2021
(51) Int. Cl.: A61L 9/12, A61L 9/04

(54) **BEDUFTUNGSVORRICHTUNG FÜR EIN FAHRZEUG**
AIR FRESHENER DEVICE FOR A VEHICLE
DISPOSITIF DE DIFFUSION DE PARFUM DANS UN VÉHICULE

(43) Veröffentlichungstag der Anmeldung: 08.03.2023
(73) Patentinhaber: Supair-Tel AG, 8152 Glattbrugg (CH)
(72) Erfinder: Guggenheim, Rudolf Heinrich, 8832 Wollerau (CH)
(74) Vertreter: E. Blum & Co. AG

(56) Entgegenhaltungen:
- CH-A2- 709 702
- DE-A1- 102011 001 637
- KR-U- 20190 000 337
- US-B2- 10 500 299

## Beschreibung

### Gebiet der Erfindung

Die Erfindung betrifft eine Beduftungsvorrichtung für ein Fahrzeug mit einem Gehäuse und einem im Gehäuse angeordneten Duftstoffbehälter. Im Weiteren umfasst die Beduftungsvorrichtung mindestens eine Koppelvorrichtung, welche am Gehäuse angeordnet ist und mindestens eine Haltevorrichtung zum Anbringen der Beduftungsvorrichtung an einer Lüftung im Innenraum eines Fahrzeuges.

### Hintergrund

Es sind Beduftungsvorrichtungen bekannt, welche im Fahrzeuginneren aufgehängt werden können und z.B. ein mit Duftstoff getränktes Material aufweisen. Solche Vorrichtungen sind kostengünstig herstellbar, ihre Beduftungswirkung kann aber ungleichmässig sein und der in der Regel am Innenrückspiegel aufgehängte Duftkörper kann sichtbehindernd sein.

Im Weiteren sind Behälter mit Duftstoff bekannt, welche an einem Lüftungsgitter im Innenraum eines Fahrzeuges befestigt werden. Der Behälter für den Duftstoff weist Einlassöffnungen auf, sodass Luft aus der Lüftung in den Behälter hineinströmt, mit dem Duftstoff in Kontakt gerät und angereichert mit Duftmolekülen den Behälter durch Auslassöffnungen verlässt. Solche Behälter können mittels eines Klemmteils am Lüftungsgitter befestigt und von diesem auch wieder entfernt werden.

US10500299B2 zeigt eine Beduftungsvorrichtung mit einem Duftstift, welcher in einem perforierten Gehäuse angeordnet ist.

DE102011001637A1 zeigt eine Beduftungsvorrichtung mit einem Behälter für einen Duftstoff. Der Behälter kann mittels einer Haltevorrichtung an einem Lüftungsschlitz im Innenraum eines Fahrzeuges fixiert werden. Die Haltevorrichtung ist aus elastischem Material.

KR20190000337U zeigt eine multifunktionale Halterung mit einer Silikonkappe.

CH709702A2 zeigt eine Beduftungsvorrichtung zur Anordnung in einem Fahrzeug. Diese umfasst einen Duftkörper, welcher mit Befestigungseinrichtungen an einem Lüftungsgitter im Inneren eines Fahrzeuges montiert werden kann.

### Darstellung der Erfindung

Es stellt sich die Aufgabe, eine Beduftungsvorrichtung bereitzustellen, welche vielseitig einsetzbar ist.

Diese Aufgabe wird durch eine Beduftungsvorrichtung für ein Fahrzeug mit den Merkmalen des Anspruchs 1 gelöst. Der Begriff "Fahrzeug" umfasst Landfahrzeuge, wie Personenkraftwagen und Lastkraftwagen, und auch Luftfahrzeuge.

Die erfindungsgemässe Beduftungsvorrichtung umfasst ein Gehäuse, in welchem ein Duftstoffbehälter angeordnet ist. Am Gehäuse ist mindestens eine Koppelvorrichtung angeordnet. Im Weiteren umfasst die Beduftungsvorrichtung mindestens eine Haltevorrichtung zum Anbringen der Beduftungsvorrichtung an einer Lüftung im Innenraum des Fahrzeuges. Die Haltevorrichtung ist mit der Koppelvorrichtung koppelbar und von dieser entkoppelbar. Somit kann die Haltevorrichtung mit dem Gehäuse gekoppelt und von diesem entkoppelt werden.

Die Möglichkeit des Entkoppelns der Haltevorrichtung vom Gehäuse hat den Vorteil, dass unterschiedliche Typen einer Haltevorrichtung am Gehäuse montiert werden können. Die Beduftungsvorrichtung kann dadurch auf unterschiedliche Lüftungen fixiert werden, sie ist flexibel einsetzbar und benutzerfreundlich. Zudem können auch abgenutzte Haltevorrichtungen in einfacher Weise durch neue Haltevorrichtungen ausgetauscht werden, ohne dass die gesamte Beduftungsvorrichtung zu ersetzen wäre.

Zudem umfasst die Beduftungsvorrichtung mindestens zwei konstruktiv unterschiedlich ausgestaltete Haltevorrichtungen, d.h. zwei unterschiedlich Typen von Haltevorrichtungen. Die mindestens zwei konstruktiv unterschiedlichen Haltevorrichtungen sind jeweils mit mindestens einer der mindestens einen Koppelvorrichtung koppelbar.

Dies kann derart ausgestaltet sein, dass jede mögliche Haltevorrichtung auf jeder Koppelvorrichtung fixiert werden kann. Es kann aber auch so ausgestaltet sein, dass ein Typ einer Haltevorrichtung nur mit einer oder mehreren bestimmten Koppelvorrichtungen, aber nicht mit sämtlichen Koppelvorrichtungen, gekoppelt werden kann. "Konstruktiv unterschiedlich" bedeutet, dass die mindestens zwei Haltevorrichtungen nicht identisch, sondern unterschiedlich ausgestaltet bzw. geformt sind. Insbesondere haften die mindestens zwei Haltevorrichtungen mittels unterschiedlichen technischen Prinzipien an der Lüftung im Innenraum des Fahrzeuges. Dies ermöglicht, die Beduftungsvorrichtung an unterschiedlichen Arten bzw. Formen von Lüftungen anzubringen. Die Beduftungsvorrichtung ist für den Benutzer flexibel einsetzbar.

Mit Vorteil können am Gehäuse mindestens drei Koppelvorrichtungen angeordnet sein. Mit jeder Koppelvorrichtung ist jeweils eine der mindestens einen Haltevorrichtung koppelbar.

Je nach Haltevorrichtung und Lüftung ist es nicht ausreichend, die Beduftungsvorrichtung lediglich mit einer einzigen Haltevorrichtung an der Lüftung zu montieren. Die Beduftungsvorrichtung kann beispielsweise gleichzeitig mit zwei Haltevorrichtungen an der Lüftung montiert sein. Zudem kann es erforderlich sein, dass je nach Typ der Haltevorrichtung diese an unterschiedlichen Positionen am Gehäuse montiert werden müssen. Deshalb sind mehrere Koppelvorrichtungen vorgesehen, um die Haltevorrichtungen an unterschiedlichen Positionen mit dem Gehäuse koppeln zu können.

Mit Vorteil sind die drei Koppelvorrichtungen voneinander beabstandet und/oder zwischen den mindestens drei Koppelvorrichtungen können Einlassöffnungen vorgesehen sind, durch welche Luft in das Gehäuse einströmen kann.

Insbesondere sind die Koppelvorrichtung und die Haltevorrichtung derart ausgestaltet, dass sie in Form einer Schnappverbindung miteinander koppeln. Eine "Schnappverbindung" ist eine Verbindung bei welcher sich die Koppelvorrichtung und die Haltevorrichtung formschlüssig fügen. Bei der Herstellung der Schnappverbindung verformt sich eines der Bauteile, insbesondere die Koppelvorrichtung, elastisch und verhakt anschliessend lösbar in Form eines Formschlusses.

Mit der Idee, unterschiedliche Haltevorrichtungen mit dem Gehäuse der Koppelvorrichtung koppeln zu können, zeigt sich eine Schnappverbindung zwischen Haltevorrichtung und Koppelvorrichtung als geeignete Lösung, damit der Benutzer die Haltevorrichtung einfach von der Koppelvorrichtung lösen und mit der Koppelvorrichtung koppeln kann.

Mit Vorteil umfasst die Koppelvorrichtung einen Rahmen und die Haltevorrichtung eine ebene Platte, derart ausgestaltet, dass die Platte der Haltevorrichtung zur Kopplung unter den Rahmen der Koppelvorrichtung schiebbar ist, insbesondere wobei die Platte mindestens eine Erhebung aufweist, welche in gekoppelter Stellung in Lücken des Rahmens eingreift.

In einer besonderen Ausführungsform weist eine der mindestens einen Haltevorrichtung eine Gummikappe auf. Insbesondere ist die Gummikappe derart ausgestaltet, dass sie zwischen zwei Lamellen der Lüftung klemmbar ist, und/oder dass die Gummikappe von der Haltevorrichtung entfernbar ist.

Die Gummikappe kann als Material Silikon, Gummi und/oder ein Elastomer, insbesondere ein thermoplastisches Elastomer, umfassen. Insbesondere kann es ein Material mit einer Härte im Bereich von 30 Shore-A bis 90 Shore-A umfassen.

Eine Gummikappe erlaubt aufgrund ihrer Elastizität ein einfaches Montieren der Beduftungsvorrichtung an der Lüftung im Fahrzeug. Die Gummikappe ist elastisch und kann sich an unterschiedliche Dimensionen einer Lüftung anpassen. Zudem erlaubt die Elastizität ein Einklemmen der Gummikappen zwischen zwei Lamellen der Lüftung.

Mit Vorteil umfasst die Beduftungsvorrichtung mindestens zwei Haltevorrichtungen mit unterschiedlich grossen Gummikappen. Der Benutzer kann somit wahlweise eine grössere oder kleinere Gummikappe montieren, je nach Ausgestaltung der Lüftung. Sind die Lamellen im Lüfter stärker voneinander beabstandet, koppelt der Benutzer eine Haltevorrichtung mit einer grösseren Gummikappe an das Gehäuse der Beduftungsvorrichtung. Sind die Lamellen jedoch weniger stark voneinander beabstandet, wählt der Benutzer eine Haltevorrichtung mit einer kleineren Gummikappe. Somit können mit einer Beduftungsvorrichtung unterschiedliche Haltevorrichtungen mit unterschiedlichen Gummikappen mitgeliefert werden, welche der Benutzer wahlweise am Gehäuse montieren kann.

Vorteilhaft umfasst die Gummikappe eine Mantelfläche, eine Grundfläche und eine der Grundfläche gegenüberliegende Oberseite. Je nach Ausgestaltung der Gummikappe kann die Oberseite Bestandteil der Mantelfläche sein. Bei der Grundfläche handelt es sich um diejenige Fläche der Gummikappe, welche im montierten Zustand zum Gehäuse der Beduftungsvorrichtung hingerichtet ist. Bei der Oberseite handelt es sich um diejenige Fläche der Gummikappe, welche im montierten Zustand vom Gehäuse der Beduftungsvorrichtung weggerichtet ist. Für das bessere Verständnis sind die definierten Flächen in den Figuren visualisiert.

Insbesondere ist an der Oberseite der Gummikappe eine Nut angeordnet und/oder an der Oberseite der Gummikappe sind zwei parallel verlaufende Oberkanten angeordnet, insbesondere wobei die zwei Oberkanten jeweils einen geradlinigen Abschnitt aufweisen.

Eine Nut führt zu einem verbesserten elastischen Verhalten der Gummikappe. Der Gummi, insbesondere die Oberkannten, können sich in den Freiraum der Nut hinein bewegen und damit ein besseres elastisches Verhalten zeigen.

Mit Vorteil weist die Randlinie der Grundfläche mindestens einen, insbesondere zwei, geradlinige Abschnitte auf. Die Grundfläche ist somit nicht ausschliesslich rund oder elliptisch geformt, sondern umfasst auch geradlinige Abschnitte. Geradlinige Abschnitte haben den Vorteil, dass die Berührungsfläche zur Lüftung, insbesondere den Lüftungslamellen grösser ist, und somit eine bessere Klemmwirkung zwischen Beduftungsvorrichtung und Lüftung erzielt werden kann.

Insbesondere umfasst die Randlinie zwei Halbkreise oder Halbellipsen, welche die zwei geradlinigen Abschnitte verbinden.

In einer weiteren Ausführungsform kann mindestens eine der Haltevorrichtung ein Spreizelement aufweisen. Eine Haltevorrichtung mit einem Spreizelement ist neben einer Haltevorrichtung mit einer Gummikappe ein weiterer, konstruktiv unterschiedlicher Typ einer Haltevorrichtung.

Insbesondere kann die Beduftungsvorrichtung sowohl mit einer Haltevorrichtung mit Spreizelement als auch mit einer Haltevorrichtung mit Gummikappe ausgeliefert werden. Dadurch stehen dem Benutzer unterschiedliche Haltevorrichtungen mit unterschiedlichen technischen Prinzipien zur Verfügung. Es ist für den Benutzer einfacher, die Beduftungsvorrichtung an unterschiedlich ausgestalteten Lüfter zu montieren, da er unterschiedliche Haltevorrichtungen zur Verfügung hat.

Insbesondere ist das Spreizelement derart ausgestaltet, dass es zwischen zwei Lamellen der Lüftung geklemmt werden kann, und/oder das Spreizelement kann mindestens zwei Spreizglieder aufweisen.

Vorteilhaft kann die Haltevorrichtung mit der Gummikappe und die Haltevorrichtung mit dem Spreizelement mit sämtlichen Koppelvorrichtungen gekoppelt werden. Dadurch erhält der Benutzer maximale Flexibilität beim Anbringen der Haltevorrichtungen an das Gehäuse.

In einer besonderen Ausführungsform sind am Gehäuse genau drei Koppelvorrichtungen angeordnet. Eine der drei Koppelvorrichtungen ist in der Mitte zwischen den beiden anderen äusseren Koppelvorrichtungen angeordnet.

In einer ersten Konfiguration ist nur die in der Mitte angeordnete Koppelvorrichtung mit einer der Haltevorrichtungen, insbesondere einer Haltevorrichtung mit einem Spreizelement, gekoppelt. In einer zweiten Konfiguration sind nur die äusseren beiden Koppelvorrichtungen mit einer der Haltevorrichtungen gekoppelt, insbesondere mit zwei Halteelementen mit jeweils einer Gummikappe. Bei der zweiten Konfiguration können Halteelemente mit kleineren oder grösseren Gummikappen gekoppelt werden.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine Beduftungsvorrichtung mit Sicht auf die Vorderseite und einem nicht eingesetzten Duftstoffbehälter;
Fig. 2 die Beduftungsvorrichtung mit Sicht auf die Rückseite, wobei zwei Gummikappen mit der Beduftungsvorrichtung gekoppelt sind;
Fig. 3 die Beduftungsvorrichtung mit Sicht auf die Rückseite, wobei ein Spreizelement mit der Beduftungsvorrichtung gekoppelt ist;
Fig. 4 eine Explosionszeichnung der Beduftungsvorrichtung ohne Duftstoffbehälter; und
Fig. 5a bis 5f eine Gummikappe in verschiedenen Ansichten.

### Weg zur Ausführung der Erfindung

### Übersicht

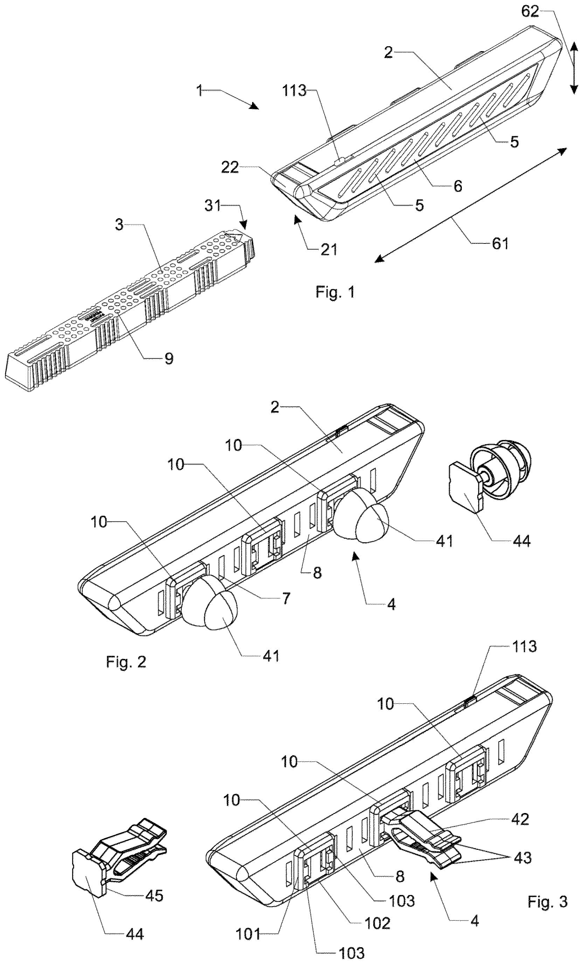

Die Figuren 1 bis 4 zeigen eine erfindungsgemässe Beduftungsvorrichtung 1 für ein Fahrzeug mit einem Gehäuse 2 und einem im Gehäuse 2 angeordneten Duftstoffbehälter 3. Der Duftstoffbehälter 3 ist auswechselbar. Er kann dem Gehäuse 2 entnommen und ein neuer Duftstoffbehälter 3 kann in das Gehäuse 2 eingesetzt werden. Die Beduftungsvorrichtung 1 weist Haltevorrichtungen 4 auf, mit welchen die Beduftungsvorrichtung 1 an einer Lüftung im Innenraum des Fahrzeuges montiert werden kann.

Die Fig. 1 zeigt die Beduftungsvorrichtung 1 mit Blick auf die Vorderseite. An der Vorderseite der Beduftungsvorrichtung 1 sind Auslassöffnungen 5 angeordnet. Die Vorderseite wird deshalb auch als Auslassseite 6 bezeichnet. Zusätzlich können auch Auslassöffnungen an einer oder an beiden Seitenwänden des Gehäuses 2 und/oder an dessen Oberseite und/oder an dessen Unterseite vorgesehen sein.

Die Fig. 2 und 3 zeigen die Beduftungsvorrichtung 1 mit Blick auf die Rückseite. An der Rückseite der Beduftungsvorrichtung 1 sind Einlassöffnungen 7 angeordnet. Die Rückseite wird deshalb auch als Einlassseite 8 bezeichnet.

Ist die Beduftungsvorrichtung 1 an der Lüftung im Innenraum des Fahrzeuges montiert, ist die Einlassseite 8 zur Lüftung gerichtet und die Auslassseite 6 ist von der Lüftung weggerichtet. Luft strömt aus der Lüftung durch die Einlassöffnungen 7, in das Gehäuse 2 und in den im Gehäuse 2 angeordneten Duftstoffbehälter 3. Die Luft kommt mit dem Duftstoff im Duftstoffbehälter 3 in Kontakt, wird mit Duftmolekülen angereichert und verlässt durch die Auslassöffnungen 5 das Gehäuse 2 der Beduftungsvorrichtung 1. Die Luft gelangt in den Innenraum des Fahrzeuges und beduftet den Innenraum.

### Duftstoffbehälter

Der Duftstoffbehälter 3, wie in Fig. 2 dargestellt, ist als auswechselbarer Duftstift vorgesehen, wobei der Duftstift als Material einen Kompositwerkstoff aus zumindest einem Polymer, insbesondere einem Elastomer, und dem Duftstoff umfasst bzw. aus einem solchen Material besteht. Dabei ist das Polymer mit dem Duftstoff angereichert und fungiert als dessen Träger. Der Duftstift weist bevorzugt mehrere Durchlassöffnungen 9 auf, durch die die aus dem Lüftungsschlitz austretende Luft strömen kann. Durch das Vorsehen der Durchlassöffnungen 9 wird die Oberfläche vergrössert, an welcher die aus dem Lüftungsschlitz austretende Luft mit dem Duftstoff in Kontakt gerät, was eine erhöhte Anreicherung der die Beduftungsvorrichtung 1 durchströmenden Luft mit Duftstoffmolekülen zur Folge hat. Die Durchlassöffnungen 9 sind vorzugsweise auf die Strömungsrichtung der Luft aus dem Lüftungsschlitz ausgerichtet. Alternativ kann der Duftstoff auch in Form eines Granulats vorgesehen sein, das in einem Käfig enthalten ist.

Wie in Fig. 1 gezeigt, verfügt das Gehäuse 2 an einem seiner Enden über eine Gehäuseöffnung 21, welche mit einem Gehäusedeckel 22 verschliessbar ist. Für den Austausch des Duftstoffbehälters 3 öffnet der Benutzer den Gehäusedeckel 22. Dieser ist über ein Drehscharnier am Gehäuse befestigt und bleibt somit auch in geöffneter Position mit dem Gehäuse 2 verbunden.

Möchte der Benutzer den neuen Duftstoffbehälter 3 einsetzen, muss er diesen in korrekter Richtung in das Gehäuse 2 bzw. in einen Schiebebehälter 11 einführen. Denn am vorderen Ende des Duftstoffbehälters 3 ist eine Vertiefung 31 vorgesehen, in welche in eingesetzter Position ein Vorsprung des Gehäuses 2 bzw. des Schiebebehälters 11 eingreift. Greifen Vertiefung 31 und Vorsprung nicht ineinander, kann der Duftstoffbehälter 3 nicht vollständig in das Gehäuse 2 eingesetzt werden und der Gehäusedeckel 22 lässt sich nicht schliessen. Hat der Benutzer den Duftstoffbehälter 3 korrekt in das Gehäuse 2 eingesetzt, kann er den Gehäusedeckel 22 schliessen. Beim Schliessen rastet der Gehäusedeckel 22 am Gehäuse 2 ein, sodass sich der Gehäusedeckel 22 nicht ungewollt öffnet.

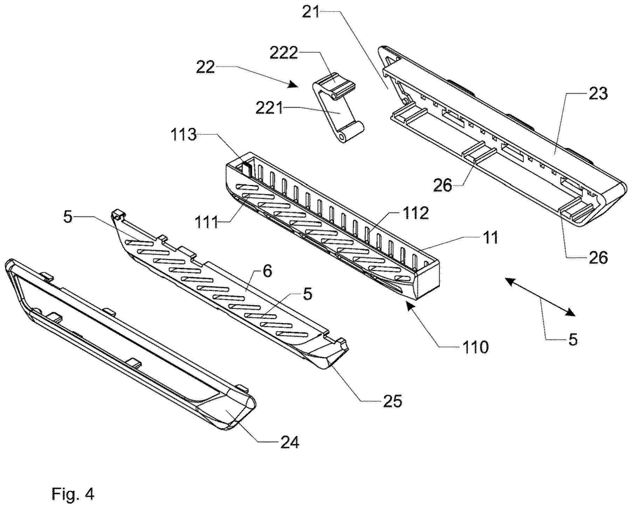

In Fig. 4 gut sichtbar ist, dass der Gehäusedeckel 22 eine gebogene Form aufweist. Der längere Abschnitt 221 des Gehäusedeckels 22 bildet das seitliche Ende des Gehäuses 2. Der kürzere Abschnitt 222 des Gehäusedeckels 22 erstreckt sich entlang eines Abschnitts der Längsseite des Gehäuses 2. Die gebogene Form des Gehäusedeckels 22 hat den Vorteil, dass die Gehäuseöffnung 21 entsprechend gross ausgestaltet ist und der Duftstoffbehälter 3 komfortabel einführbar ist.

### Ausgestaltung der Auslassseite

Wie in Fig. 1 ersichtlich, weist das Gehäuse 2 und somit auch die Auslassseite 6 eine längliche Form mit einer Längsseite 61 und einer Kurzseite 62 auf. Auf der Auslassseite 6 sind mehrere Auslassöffnungen 5 angeordnet. Sie weisen jeweils die Form eines länglichen Schlitzes auf und sind parallel und äquidistant zueinander angeordnet.

Die Auslassöffnungen 5 sind jeweils schräg zur Längsseite 61 und schräg zur Kurzseite 62 angeordnet. Mit anderen Worten sind die Auslassöffnungen 5 weder parallel zur Längsseite 61, noch parallel zur Kurzseite 62 angeordnet. Schräg bedeutet in der vorliegenden Ausführungsform, dass die als Schlitze ausgestalteten Auslassöffnungen 5 in einem Winkel von 45° zur Längsseite 61 und in einem Winkel von 45° zur Kurzseite 62 angeordnet sind. Die Auslassöffnungen 5 erstrecken sich jeweils annähernd über die gesamte Länge der Kurzseite 62.

### Haltevorrichtungen

An der Einlassseite 8 sind drei Koppelvorrichtungen 10 angeordnet. Mit jeder der drei Koppelvorrichtungen 10 kann jeweils eine Haltevorrichtung 4 gekoppelt werden. Die drei Koppelvorrichtungen 10 sind voneinander beabstandet und zwischen ihnen sind Einlassöffnungen 7 angeordnet.

In Fig. 2 ist eine erste Variante dargestellt, die Beduftungsvorrichtung 1 an die Lüftung des Fahrzeuges zu montieren. Mit den äusseren Koppelvorrichtungen 10 sind jeweils eine Haltevorrichtung 4 mit einer Gummikappe 41 gekoppelt. Die mittlere Koppelvorrichtung 10 ist freigelassen und mit keiner Haltevorrichtung 4 gekoppelt.

Die Gummikappen 41 sind aus einem elastischen Material und derart ausgestaltet, dass sie zwischen zwei Lamellen der Lüftung im Fahrzeug geklemmt werden können. Die Haltevorrichtungen 4 mit den Gummikappen 41 können somit haltend in einen Lüftungsschlitz im Fahrzeuginnenraum eingebracht werden. Das heisst, die Dimensionen der Gummikappen 41 sind in Abhängigkeit von den Dimensionen, insbesondere der Breite, des Lüftungsschlitzes derart gewählt, dass die Beduftungsvorrichtung 1 an den Lüftungsschlitz angebracht werden kann, indem die Gummikappen 41 in den Lüftungsschlitz eingeführt/eingebracht werden und dort von diesem klemmend gehalten werden. Die Gummikappen 41 sind auf die Haltevorrichtungen 4 aufsteckbar. Dadurch können die Gummikappen 41 in einfacher Weise ausgewechselt werden.

Das Gehäuse 2 ist bevorzugt länglich ausgeführt. Ist es an einem Lüftungsschlitz montiert, erstreckt es sich entlang des Lüftungsschlitzes. Beide Haltevorrichtungen 4 sind in demselben Lüftungsschlitz angeordnet. Selbstverständlich ist es auch möglich, das Gehäuse 2 quer zu den Lüftungsschlitzen anzuordnen, wobei die Haltevorrichtungen 4 dann in unterschiedliche Lüftungsschlitze eingebracht sind.

In Fig. 3 ist eine zweite Variante dargestellt, die Beduftungsvorrichtung 1 an die Lüftung des Fahrzeuges zu montieren. Die äusseren Koppelvorrichtungen 10 sind freigelassen und mit keiner Haltevorrichtung 4 gekoppelt. Lediglich an der mittleren Koppelvorrichtung 10 ist eine Haltevorrichtung 4 angeordnet, welche ein Spreizelement 42 aufweist. Das Spreizelement 42 umfasst zwei Spreizglieder 43 und kann wie die Gummikappen 41 zwischen zwei Lamellen der Lüftung des Fahrzeuges geklemmt werden.

Die Haltevorrichtung 4 mit der Gummikappe 41 und die Haltevorrichtung 4 mit dem Spreizelement 42 stellen unterschiedliche Typen von Haltevorrichtungen 4 dar, welche konstruktiv unterschiedlich ausgestaltet sind.

Alle drei Koppelvorrichtungen 10 sind identisch ausgestaltet. D.h. sowohl die Haltevorrichtungen 4 mit den Gummikappen 41 als auch die Haltevorrichtung 4 mit dem Spreizelement 42 können mit sämtlichen drei Koppelvorrichtungen gekoppelt und somit untereinander ausgetauscht werden. Anspruchsgemäss kann somit jede der Haltevorrichtungen 4 mit einer der mindestens einen Koppelvorrichtung 10 gekoppelt werden.

Wie in den Fig. 2 und 3 ersichtlich, koppeln die Koppelvorrichtungen 10 mit den Haltevorrichtungen 4 in Form einer Schnappverbindung. Die Koppelvorrichtungen 10 weisen einen quadratischen Rahmen 101 auf. Der Rahmen 101 ist auf drei Seiten geschlossen und weist an seiner Unterseite eine Rahmenöffnung 102 auf. Im Rahmen 101 sind zudem auf der linken und auf der rechten Seite jeweils zwei Lücken 103 angeordnet.

Die Haltevorrichtung 4 umfasst eine ebene, quadratische Platte 44 mit welcher je nach Typ der Haltevorrichtung 4 entweder die Gummikappen 41 oder das Spreizelement 42 verbunden sind. An der Platte 44 sind Erhebungen 45 angeordnet, welche in eingeschnappter Position in die Lücken 103 des Rahmens 101 der Koppelvorrichtung 10 eingreifen können.

Ein Benutzer kann die Haltevorrichtung 4 mit der Koppelvorrichtung 10 koppeln, indem er die Platte 44 der Haltevorrichtung 4 von unten durch die Rahmenöffnung 102 unter den Rahmen 101 schiebt. Dabei deformiert sich der Rahmen 101 elastisch, weil die Erhebungen 45 auf den Rahmen 101 drücken. Der Benutzer schiebt die Platte 44 weiter, bis die Erhebungen 45 in den Lücken 103 erscheinen. Der Rahmen 101 deformiert sich zurück, und die Platte 44 ist im Rahmen 101 eingeschnappt. Haltevorrichtung 4 und Koppelvorrichtung 10 sind miteinander gekoppelt.

Möchte ein Benutzer die Haltevorrichtung 4 von der Koppelvorrichtung 10 entkoppeln, bewegt er die Haltevorrichtung 4 nach unten, sodass die Platte 44 durch die Rahmenöffnung 102 aus dem Rahmen 101 geführt wird. Der Benutzer muss für diese Bewegung, d.h. für die Aufhebung der Schnappverbindung eine gewisse Kraft aufbringen, da sich der Rahmen 101 aufgrund der Erhebungen 45 erneut elastisch deformiert.

Wie bereits beschrieben, stehen dem Benutzer unterschiedliche Varianten zur Verfügung, um die Beduftungsvorrichtung 1 an die Lüftung im Fahrzeug zu montieren. Einerseits kann der Benutzer zur Montage die Haltevorrichtungen 4 mit den Gummikappen 41, andererseits die Haltevorrichtung 4 mit dem Spreizelement 42 verwenden. Der Benutzer verfügt somit über ein Kit, welches einerseits das Gehäuse 2 mit dem Duftstoffbehälter 3 und andererseits die unterschiedlichen Typen von Haltevorrichtungen 4 umfasst. Zusätzlich können im Kit auch unterschiedlich grosse Gummikappen 41 vorhanden sein. D.h. dem Benutzer stehen in diesem Fall insgesamt drei Varianten zur Verfügung, um die Beduftungsvorrichtung 1 an der Lüftung im Innenraum des Fahrzeuges zu montieren. Die Montage erfolgt mittels den kleineren Gummikappen 41, mittels den grösseren Gummikappen 41 oder mittels des Spreizelements 42. Der Benutzer wählt aus den drei Varianten diejenige aus, welche sich in Abhängigkeit der Ausgestaltung der Lüftung am besten eignet, um die Beduftungsvorrichtung 1 zu montieren.

### Schiebevorrichtung

Fig. 4 zeigt eine Explosionsdarstellung der Einzelteile der Beduftungsvorrichtung 1. Der Duftstoffbehälter 3 ist nicht sichtbar. Das Gehäuse 2 umfasst eine rückseitige Gehäusehülle 23, einen frontseitigen Gehäuserahmen 24, welcher eine frontseitige Gehäuseabdeckung 25 einrahmt. Die Gehäuseabdeckung 25 umfasst die bereits erwähnten Auslassöffnungen 5 an der Auslassseite 6. Ebenfalls gezeigt ist der bereits erwähnte Gehäusedeckel 22, welcher die Gehäuseöffnung 21 verschliessen kann.

Innerhalb des Gehäuses 2 ist eine Schiebevorrichtung mit einem Schiebebehälter 11 angeordnet. Der Schiebebehälter umfasst einen Boden 110, eine vordere Wand mit schrägen Auslassöffnungen 111 und ein rückseitige Wand mit vertikalen Einlassöffnungen 112. Zwischen den Wänden des Schiebebehälters 11 ist Raum vorhanden, sodass der Duftstoffbehälter 3 innerhalb des Schiebebehälters 11 angeordnet werden kann.

An der Innenseite des Gehäuses 2 sind Stege 26 vorhanden, auf welchen sich der Schiebebehälter 11 bodenseitig abstützt oder welche er mit seinen seitlichen Wänden berührt. Dadurch ist die Kontaktfläche zwischen dem Gehäuse 2 und dem Schiebebehälter 11 möglichst klein gehalten. Der Schiebebehälter 11 lässt sich innerhalb des Gehäuses 2 leicht verschieben.

Zur Bewegung des Schiebebehälters 11 ist an seiner Oberseite ein plattenförmiger Griff 113 angeordnet, welcher durch die Oberseite des Gehäuses 2 aus diesem herausragt. Der Griff 113 ragt somit weder durch die Einlassseite 8, noch durch die Auslassseite 6. Der Griff 113 ist auch in Fig. 1 sichtbar. Durch Betätigung des Griffes 113 kann der Benutzer die Schiebevorrichtung 11 relativ zum Gehäuse 2 in Längsrichtung verschieben. Der Schiebebehälter 11 wird dabei in Richtung des in Fig. 4 gezeigten Doppelpfeils 114 hin- und her bewegt.

Da der Duftstoffbehälter 3 innerhalb des Schiebebehälters 11 angeordnet ist und das Gehäuse 2 nicht berührt, bewegt sich der Duftstoffbehälter 3 in gleicherweise wie der Schiebebehälter 11 relativ zum Gehäuse 2. Dadurch reibt sich der Duftstoffbehälter 3 während der Bewegung nicht mit dem Gehäuse 2. Reibung entsteht lediglich zwischen dem Schiebebehälter 11 und den Stegen 26 des Gehäuses 2. Die Bewegung erfolgt somit kontrolliert, mit nur geringfügiger Reibung und ist unabhängig davon, ob der Duftstoffbehälter 3 vom Benutzer sauber im Schiebebehälter 11 eingesetzt wurde.

Mittels der Bewegung des Schiebebehälters 11 kann der Benutzer die Einlassöffnungen 7 und die Auslassöffnungen 5 öffnen und schliessen. Im geschlossenen Zustand verdecken die Streben des Schiebebehälters 11 die Einlassöffnungen 7 und die Auslassöffnungen 5. Luft kann die Beduftungsvorrichtung 1 nicht durchströmen. Im offenen Zustand sind die Auslassöffnungen 111 des Schiebebehälters 11 deckungsgleich mit den Auslassöffnungen 5 des Gehäuses 2 und die Einlassöffnungen 112 des Schiebebehälters 11 sind deckungsgleich mit den Einlassöffnungen 7 des Gehäuses 2. Luft kann durch die Beduftungsvorrichtung 1 strömen und mit Duftstoffmolekülen des Duftstoffbehälters 3 angereichert werden.

### Ausgestaltung der Gummikappen

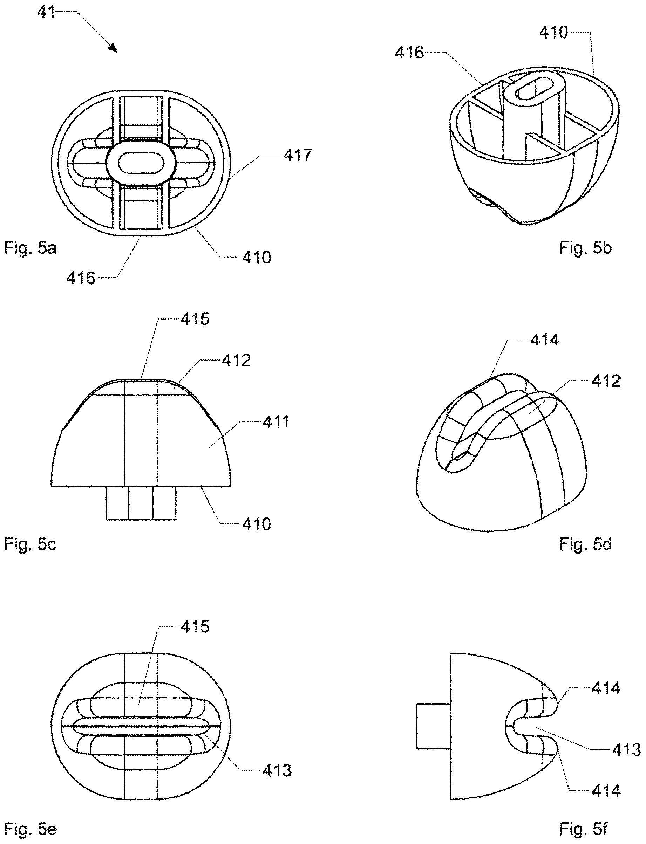

Fig. 5a bis 5f zeigen die an den Haltevorrichtungen 4 angeordneten Gummikappen 41 in detaillierter Ansicht und aus verschiedenen Perspektiven. Die Gummikappe 41 weist eine Grundfläche 410, eine Mantelfläche 411 und eine der Grundfläche 410 gegenüberliegende Oberseite 412 auf. Da die in Fig. 6 dargestellte Gummikappe 41 eine abgerundete Oberseite 412 aufweist, bildet die Oberseite 412 einen Bestandteil der Mantelfläche 411.

Bei der Grundfläche handelt es sich um diejenige Fläche der Gummikappe, welche im montierten Zustand zum Gehäuse der Beduftungsvorrichtung hingerichtet ist. Bei der Oberseite handelt es sich um diejenige Fläche der Gummikappe, welche im montierten Zustand vom Gehäuse der Beduftungsvorrichtung weggerichtet ist.

An der Oberseite 412 der Gummikappe 41 ist eine längliche Nut 413 angeordnet. Die Nut 413 trennt die Oberseite 412 in zwei parallel verlaufende Oberkanten 414. Diese Oberkanten 414 weisen jeweils einen geradlinigen Abschnitt 415 auf.

Die Grundfläche 410 der Gummikappe 41 weist einen Umfang bzw. eine Randlinie mit zwei geradlinigen Abschnitten 416 auf. Diese beiden geradlinigen Abschnitte 416 werden durch zwei Halbkreise 417 miteinander verbunden. Die Grundfläche 410 ähnelt somit einer elliptischen Form, wobei es sich aber gerade nicht um eine elliptische Form handelt, weil die Randlinie geradlinige Abschnitte 416 aufweist.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Beduftungsvorrichtung (1) für ein Fahrzeug aufweisend
- ein Gehäuse (2),
- ein im Gehäuse (2) angeordneter Duftstoffbehälter (3),
- mindestens eine am Gehäuse (2) angeordnete Koppelvorrichtung (10),
- mindestens eine Haltevorrichtung (4) zum Anbringen der Beduftungsvorrichtung (1) an einer Lüftung im Innenraum des Fahrzeuges,
wobei die Haltevorrichtung (4) mit der Koppelvorrichtung (10) koppelbar und von dieser entkoppelbar ist,
**dadurch gekennzeichnet, dass** die Beduftungsvorrichtung mindestens zwei konstruktiv unterschiedlich ausgestaltete Haltevorrichtungen (4) umfasst, wobei beide Haltevorrichtungen (4) mit einer der mindestens einen Koppelvorrichtung (10) koppelbar sind.

2. Beduftungsvorrichtung nach Anspruch 1, wobei am Gehäuse (2) mindestens drei Koppelvorrichtungen (10) angeordnet sind, mit welchen jeweils eine der Haltevorrichtungen (4) koppelbar ist, insbesondere
wobei die drei Koppelvorrichtungen (10) voneinander beabstandet sind, und/oder
wobei zwischen den mindestens drei Koppelvorrichtungen (10) Einlassöffnungen (7) vorgesehen sind, durch welche Luft in das Gehäuse (2) einströmen kann.

3. Beduftungsvorrichtung nach einem der vorangehenden Ansprüche, wobei die Koppelvorrichtung (10) und die Haltevorrichtungen (4) derart ausgestaltet sind, dass sie in Form einer Schnappverbindung miteinander koppeln.

4. Beduftungsvorrichtung nach einem der vorangehenden Ansprüche, wobei die Koppelvorrichtung (10) einen Rahmen (101) und die Haltevorrichtungen (4) eine ebene Platte (44) aufweisen, derart ausgestaltet, dass die Platte (44) der Haltevorrichtungen (4) zur Kopplung unter den Rahmen (101) der Koppelvorrichtung (10) schiebbar ist,
insbesondere wobei die Platte (44) mindestens eine Erhebung (45) aufweist, welche in gekoppelter Stellung in Lücken (103) des Rahmens eingreift.

5. Beduftungsvorrichtung nach einem der vorangehenden Ansprüche, wobei mindestens eine der Haltevorrichtungen (4) eine Gummikappe (41) aufweist, insbesondere
- wobei die Gummikappe (41) derart ausgestaltet ist, dass sie zwischen zwei Lamellen der Lüftung klemmbar ist, und/oder
- wobei die Gummikappe (41) von der Haltevorrichtung (4) entfernbar ist.

6. Beduftungsvorrichtung nach Anspruch 5, wobei die Beduftungsvorrichtung (1) mindestens zwei Haltevorrichtungen (4) mit den Gummikappen (41) aufweist, wobei die mindestens zwei Haltevorrichtungen (4) Gummikappen (41) unterschiedlicher Grössen aufweisen.

7. Beduftungsvorrichtung nach Anspruch 5 oder 6, wobei die Gummikappe (41) eine Mantelfläche (411), eine Grundfläche (410) und eine der Grundfläche (410) gegenüberliegende Oberseite (412) aufweist.

8. Beduftungsvorrichtung nach Anspruch 7, wobei an der Oberseite (412) der Gummikappe (41) eine Nut (413) angeordnet ist und/oder wobei an der Oberseite (412) der Gummikappe (41) zwei parallel verlaufende Oberkanten (414) angeordnet sind, insbesondere wobei die zwei Oberkanten (414) jeweils einen geradlinigen Abschnitt (415) aufweisen.

9. Beduftungsvorrichtung nach Anspruch 7 oder 8, wobei eine Randlinie der Grundfläche (410) mindestens einen, insbesondere zwei geradlinige Abschnitte (416) aufweist,
insbesondere wobei die Randlinie zwei Halbkreise (417) oder Halbellipsen umfasst, welche die zwei geradlinigen Abschnitte (416) verbinden.

10. Beduftungsvorrichtung nach einem der vorangehenden Ansprüche, wobei mindestens eine der Haltevorrichtungen (4) ein Spreizelement (42) aufweist, insbesondere
- wobei das Spreizelement (42) derart ausgestaltet ist, dass es zwischen zwei Lamellen der Lüftung klemmbar ist, und/oder
- wobei das Spreizelement (42) mindestens zwei Spreizglieder (43) aufweist.

11. Beduftungsvorrichtung nach einem der Ansprüche 5 bis 9 und nach Anspruch 10, wobei die Haltevorrichtung (4) mit der Gummikappe (41) und die Haltevorrichtung (4) mit dem Spreizelement (42) auf sämtliche Koppelvorrichtungen (4) koppelbar sind.

12. Beduftungsvorrichtung nach einem der vorangehenden Ansprüche, wobei am Gehäuse (2) genau drei Koppelvorrichtungen (10) angeordnet sind und eine der drei Koppelvorrichtungen (10) mittig zwischen den beiden anderen äusseren Koppelvorrichtungen (10) angeordnet ist,
insbesondere wobei
- nur die mittig angeordnete Koppelvorrichtung (10) mit einer der Haltevorrichtungen (4) gekoppelt ist, oder
- nur die äusseren beiden Koppelvorrichtungen (10) mit einer der Haltevorrichtungen (4) gekoppelt ist.

13. Beduftungsvorrichtung nach Anspruch 11 und 12, wobei
- die mittige Koppelvorrichtung (10) mit der Haltevorrichtung (4) mit dem Spreizelement (42) gekoppelt ist, und insbesondere an den beiden äusseren Koppelvorrichtungen (10) keine Haltevorrichtung (4) gekoppelt ist, oder
- die beiden äusseren Koppelvorrichtungen (10) jeweils mit einer der Haltevorrichtungen (4) mit der Gummikappe (41) gekoppelt ist, und insbesondere an der mittleren Koppelvorrichtung (10) keine Haltevorrichtung (4) gekoppelt ist.

## Claims

1. Scenting device (1) for a vehicle comprising
- a housing (2),
- a scent container (3) arranged in the housing (2),
- at least one coupling device (10) arranged on the housing (2),
- at least one holding device (4) for attaching the scenting device (1) to a ventilation system in the interior of the vehicle,
wherein the holding device (4) can be coupled to and decoupled from the coupling device (10),
**characterised in that** the scenting device comprises at least two differently designed holding devices (4), wherein both holding devices (4) can be coupled to one of the at least one coupling device (10).

2. Scenting device according to claim 1, wherein at least three coupling devices (10) are arranged on the housing (2), to each of which one of the holding devices (4) can be coupled, in particular
wherein the three coupling devices (10) are spaced apart from one another, and/or
wherein inlet openings (7) are provided between the at least three coupling devices (10), through which air can flow into the housing (2).

3. Scenting device according to one of the preceding claims, wherein the coupling device (10) and the holding devices (4) are designed in such a way that they couple to one another in the form of a snap connection.

4. Scenting device according to one of the preceding claims, wherein the coupling device (10) has a frame (101) and the holding devices (4) have a flat plate (44), designed in such a way that the plate (44) of the holding devices (4) can be pushed under the frame (101) of the coupling device (10) for coupling,
in particular wherein the plate (44) has at least one elevation (45) which engages in gaps (103) of the frame in the coupled position.

5. Scenting device according to one of the preceding claims, wherein at least one of the holding devices (4) comprises a rubber cap (41), in particular
- wherein the rubber cap (41) is designed such that it can be clamped between two louvres of the ventilation, and/or
- wherein the rubber cap (41) can be removed from the holding device (4).

6. Scenting device according to claim 5,
wherein the scenting device (1) has at least two holding devices (4) with the rubber caps (41), wherein the at least two holding devices (4) have rubber caps (41) of different sizes.

7. Scenting device according to claim 5 or 6, wherein the rubber cap (41) has a lateral surface (411), a base surface (410) and an upper surface (412) opposite the base surface (410).

8. Scenting device according to claim 7,
wherein a groove (413) is arranged on the upper side (412) of the rubber cap (41) and/or wherein two parallel upper edges (414) are arranged on the upper side (412) of the rubber cap (41), in particular wherein the two upper edges (414) each have a rectilinear section (415).

9. Scenting device according to claim 7 or 8, wherein an edge line of the base surface (410) has at least one, in particular two rectilinear sections (416),
in particular wherein the edge line comprises two semicircles (417) or half ellipses which connect the two rectilinear sections (416).

10. Scenting device according to one of the preceding claims, wherein at least one of the holding devices (4) comprises a spreading element (42), in particular
- wherein the spreading element (42) is configured such that it can be clamped between two louvres of the ventilation, and/or
- wherein the spreading element (42) has at least two spreading members (43).

11. Scenting device according to one of claims 5 to 9 and according to claim 10, wherein the holding device (4) can be coupled to the rubber cap (41) and the holding device (4) can be coupled to the spreading element (42) on all coupling devices (4).

12. Scenting device according to one of the preceding claims, wherein exactly three coupling devices (10) are arranged on the housing (2) and one of the three coupling devices (10) is arranged centrally between the other two outer coupling devices (10),
in particular wherein
- only the centrally arranged coupling device (10) is coupled to one of the holding devices (4), or
- only the two outer coupling devices (10) are coupled to one of the holding devices (4).

13. Scenting device according to claims 11 and 12, wherein
- the central coupling device (10) is coupled to the holding device (4) with the spreading element (42), and in particular no holding device (4) is coupled to the two outer coupling devices (10), or
- the two outer coupling devices (10) are each coupled to one of the holding devices (4) with the rubber cap (41), and in particular no holding device (4) is coupled to the centre coupling device (10).

## Revendications

1. Dispositif de parfumage (1) pour un véhicule comprenant
- un boîtier (2)
- un réservoir de parfum (3) placé dans le boîtier (2),
- au moins un dispositif de couplage (10) disposé sur le boîtier (2),
- au moins un dispositif de support (4) pour fixer le dispositif de parfumage (1) à un système de ventilation à l'intérieur du véhicule,
dans lequel le dispositif de support (4) peut être couplé et découplé du dispositif de couplage (10), **caractérisé en ce que** le dispositif de parfumage comprend au moins deux dispositifs de support (4) de construction différente, les deux dispositifs de support (4) pouvant être couplés à l'un d'au moins un dispositif de couplage (10).

2. Dispositif de parfumage selon la revendication 1, dans lequel au moins trois dispositifs de couplage (10) sont disposés sur le boîtier (2), à chacun desquels l'un des dispositifs de support (4) peut être couplé, en particulier
dans lequel les trois dispositifs de couplage (10) sont espacés les uns des autres, et/ou dans lequel des ouvertures d'entrée (7) sont prévues entre les trois dispositifs de couplage (10), par lesquelles l'air peut s'écouler dans le boîtier (2).

3. Dispositif de parfumage selon l'une des revendications précédentes, dans lequel le dispositif de couplage (10) et les dispositifs de support (4) sont conçus de manière à se coupler l'un à l'autre sous la forme d'un raccord rapide.

4. Dispositif de parfumage selon l'une des revendications précédentes, dans lequel le dispositif de couplage (10) a un cadre (101) et les dispositifs de support (4) ont une plaque plate (44), conçue de manière à ce que la plaque (44) des dispositifs de support (4) puisse être poussée sous le cadre (101) du dispositif de couplage (10) pour le couplage,
en particulier, la plaque (44) comportant au moins une élévation (45) qui s'engage dans les interstices (103) du cadre en position de couplage.

5. Dispositif de parfumage selon l'une des revendications précédentes, dans lequel au moins un des dispositifs de support (4) comprend un capuchon en caoutchouc (41), en particulier
- dans lequel le capuchon en caoutchouc (41) est conçu de manière à pouvoir être serré entre deux grilles d'aération, et/ou
- dans lequel le capuchon en caoutchouc (41) peut être retiré du dispositif de support (4).

6. Dispositif de parfumage selon la revendication 5, dans lequel le dispositif de parfumage (1) a au moins deux dispositifs de support (4) avec les capuchons en caoutchouc (41), dans lequel les au moins deux dispositifs de support (4) ont des capuchons en caoutchouc (41) de tailles différentes.

7. Dispositif de parfumage selon la revendication 5 ou 6, dans lequel le capuchon en caoutchouc (41) a une surface latérale (411), une surface de base (410) et une surface supérieure (412) opposée à la surface de base (410) .

8. Dispositif de parfumage selon la revendication 7, dans lequel une rainure (413) est disposée sur la face supérieure (412) du capuchon en caoutchouc (41) et/ou dans lequel deux bords supérieurs parallèles (414) sont disposés sur la face supérieure (412) du capuchon en caoutchouc (41), en particulier dans lequel les deux bords supérieurs (414) ont chacun une section rectiligne (415).

9. Dispositif de parfumage selon la revendication 7 ou 8, dans lequel une ligne de bord de la surface de base (410) a au moins une, en particulier deux sections rectilignes (416),
en particulier, dans lequel la ligne de bord comprend deux demi-cercles (417) ou demi ellipses qui relient les deux sections rectilignes (416).

10. Dispositif de parfumage selon l'une des revendications précédentes, dans lequel au moins un des dispositifs de support (4) comprend un élément d'écartement (42), en particulier
- dans lequel l'élément d'écartement (42) est configuré de manière à pouvoir être serré entre deux grilles de ventilation, et/ou
- dans lequel l'élément d'écartement (42) comporte au moins deux éléments d'écartement (43).

11. Dispositif de parfumage selon l'une des revendications 5 à 9 et selon la revendication 10, dans lequel le dispositif de support (4) peut être couplé au capuchon en caoutchouc (41) et le dispositif de support (4) peut être couplé à l'élément d'écartement (42) sur tous les dispositifs de couplage (4).

12. Dispositif de parfumage selon l'une des revendications précédentes, dans lequel exactement trois dispositifs de couplage (10) sont disposés sur le boîtier (2) et l'un des trois dispositifs de couplage (10) est disposé au centre entre les deux autres dispositifs de couplage extérieurs (10),
en particulier, dans lequel
- seul le dispositif de couplage (10) disposé au centre est couplé à l'un des dispositifs de support (4), ou
- seuls les deux dispositifs de couplage extérieurs (10) sont couplés à l'un des dispositifs de support (4) .

13. Dispositif de parfumage selon la revendication 11 et 12, dans lequel
- le dispositif de couplage (10) disposé au centre est couplé au dispositif de support (4) avec l'élément d'écartement (42), et en particulier
aucun dispositif de support (4) n'est couplé aux deux dispositifs de couplage extérieurs (10), ou
les deux dispositifs de couplage extérieurs (10) sont chacun couplé à un dispositif de support (4) au capuchon en caoutchouc (41), et en particulier aucun dispositif de support (4) n'est couplé au dispositif de couplage disposé au centre (10).
